# EUROPEAN PATENT SPECIFICATION

(11) **EP 4 538 690 B1**
(45) Date of publication and mention of the grant of the patent: **24.06.2026**
(21) Application number: 23202537.9
(22) Date of filing: 09.10.2023
(51) Int. Cl.: G01N 27/20, G01N 33/44

(54) **METHOD OF DETERMINING COMPATIBILITY BETWEEN POLYMERIC INSULATION MATERIAL AND A FOREIGN MATERIAL**
VERFAHREN ZUR BESTIMMUNG DER KOMPATIBILITÄT ZWISCHEN EINEM POLYMERISOLIERMATERIAL UND EINEM FREMDMATERIAL
PROCÉDÉ DE DÉTERMINATION DE COMPATIBILITÉ ENTRE UN MATÉRIAU D'ISOLATION POLYMÈRE ET UN MATÉRIAU ÉTRANGER

(43) Date of publication of application: 16.04.2025
(73) Proprietor: NKT HV Cables AB, 371 60 Lyckeby (SE)
(72) Inventor: ABBASI, Amirhossein, Karlskrona (SE); HOANG, Anh, Västerås (SE)
(74) Representative: Kransell & Wennborg KB

(56) References cited:
- CN-A- 107 728 029
- FR-A1- 2 607 254
- US-A1- 2017 285 093

## Description

### TECHNICAL FIELD

The present disclosure generally relates to high voltage power cables, in particular a method of determining compatibility between polymeric insulation materials and a foreign material.

### BACKGROUND

Power cables have an insulation system which electrically insulate the conductor. The insulation system typically comprises an inner semiconducting layer, an insulating layer, and an outer semiconducting layer. The DC performance of the insulation system is governed by the conductivity of the insulation layer. The insulation layer may be in direct or indirect contact with other layers in the power cable, or with materials during handling. There is a risk that the insulation layer becomes contaminated by one or more of these layers or from the materials contacting the insulation system during handling. This may result in that the conductivity of the insulation material increases, which can lead to an increased risk of failure in the power cable during operation and/or testing.

FR 2 607 254 A1 relates to a method for characterising an electrically insulating polymer film, enabling evaluation of the film's insulating properties, in particular for detecting insulation defects or assessing various characteristics related to its insulating nature, especially its ability to withstand electrical stresses without deterioration of its insulating properties over time, as well as its surface properties (adhesion capability, wettability, ability to retain electrostatic charges).

### SUMMARY

In view of the above, an object of the present disclosure is to provide a method which solves or at least mitigates the problems of the prior art. There is hence provided a method of determining compatibility between a polymeric electrical insulation material for an insulation layer of a high voltage power cable and a foreign material as defined in claim 1. The method comprising: a) preparing a test sample from A) an insulation plate consisting of the polymeric electrical insulation material, and B) the foreign material, b) applying a voltage over the test sample, c) determining a conductivity of the test sample based on measurements taken during step b), d) comparing the conductivity of the test sample with a reference conductivity, and e) concluding whether the foreign material is compatible with the polymeric electrical insulation material based on the comparison in step d).

The term "compatibility" is to be understood to mean that the inherent conductivity of the polymeric electrical insulation material is left sufficiently unaffected by the presence of the foreign material after preparation of the test sample, under the test condition. The foreign material should thus not contaminate the polymeric electrical insulation material to a degree higher than an acceptable amount from the reference conductivity.

The method enables selecting materials for a power cable which are compatible with the polymeric electrical insulation material, forming part of the insulation system, without having to do testing by building an actual test high voltage power cable for this purpose. A low conductivity of the polymeric electrical insulation material, as required for the application, may thus be ensured for the high voltage power cable in a fast, simple, and inexpensive way.

The polymeric electrical insulation material may for example be polyethylene based, such as crosslinked polyethylene (XLPE), polypropylene-based, ethylene propylene diene monomer rubber (EPDM), ethylene propylene rubber (EPR) or any other suitable extrudable polymeric electrical insulation material.

According to one embodiment step e) involves concluding that the foreign material is compatible with the polymeric electrical insulation material in case the conductivity deviates with at most a predetermined amount from the reference conductivity, and otherwise concluding that the foreign material is non-compatible with the polymeric electrical insulation material.

The deviation of the conductivity is with regards to the conductivity being larger than the reference conductivity with the predetermined amount.

According to one embodiment, steps b) and c) are performed in a conductivity cell.

According to one embodiment in step a) the insulation plate is set in direct contact with the foreign material.

According to one embodiment step a) further involves pressing together the insulation plate and the foreign material in a press moulding machine.

According to one embodiment step a) involves heat treating the insulation plate and the foreign material when they are in direct contact.

According to one embodiment the heat treatment involves heating at a temperature in the range of 70-110°C. The temperature may for example be selected based on the maximum allowed operational temperature of a high voltage power cable using the polymeric electrical insulation material as material for the insulation layer of its insulation system. For example, if the maximum allowed temperature is 70°C, 80°C, or 90°C then the temperature in the heat treatment step is 70°C, 80°C, or 90°C, respectively.

According to one embodiment the heat treatment involves subjecting the insulation plate and the foreign material to temperature cycles between room temperature and said temperature in the range of 70-110°C. This simulates load changes during operation of a high voltage power cable.

According to one embodiment the reference conductivity is the conductivity of a non-contaminated reference sample of the polymeric electrical insulation material.

According to one embodiment an electric field over the test sample in step b) is in a range of 15-50 kV/mm.

According to one embodiment the insulation plate has a thickness in a range of 0.5-2 mm. For example, the insulation plate may have a thickness in the range 0.8-1.5 mm.

According to one embodiment the foreign material is one of a polymeric material, a lubricating material, a metal, and a semiconductive material.

According to one embodiment the foreign material is a conductor tape or a swelling tape. The swelling tape is a water swellable tape, typically comprising a superabsorbent polymer.

According to one embodiment the polymeric electrical insulation material is a DC insulation material.

According to one embodiment the DC insulation material is designed for voltages greater than 300 kV.

Generally, all terms used in the claims are to be interpreted according to their ordinary meaning in the technical field, unless explicitly defined otherwise herein. All references to "a/an/the element, apparatus, component, means", etc. are to be interpreted openly as referring to at least one instance of the element, apparatus, component, means, etc., unless explicitly stated otherwise.

### BRIEF DESCRIPTION OF THE DRAWINGS

The specific embodiments of the inventive concept will now be described, by way of example, with reference to the accompanying drawings, in which:
Fig. 1 schematically shows an electrode system for conductivity measurements; and
Fig. 2 is a flowchart of a method of determining compatibility between a polymeric electrical insulation material for an insulation layer of a high voltage power cable and a foreign material.

### DETAILED DESCRIPTION

The inventive concept will now be described more fully hereinafter with reference to the accompanying drawings, in which exemplifying embodiments are shown. The inventive concept may, however, be embodied in many different forms and should not be construed as limited to the embodiments set forth herein; rather, these embodiments are provided by way of example so that this disclosure will be thorough and complete, and will fully convey the scope of the inventive concept to those skilled in the art. Like numbers refer to like elements throughout the description.

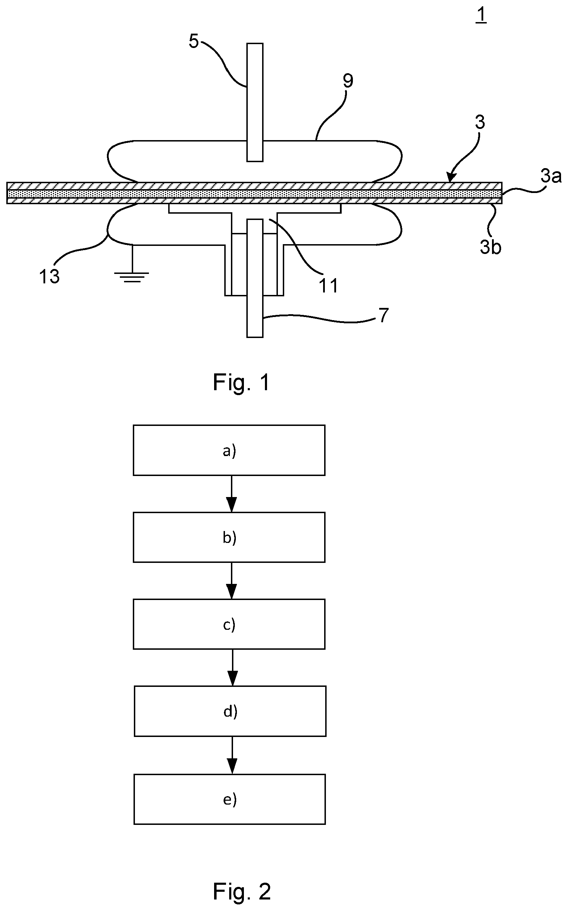

Fig. 1 schematically shows an electrode system, or conductivity cell, 1 in an experimental setup to determine the conductivity of a test sample 3. Based on the determined conductivity, the compatibility of a foreign material and a polymeric electrical insulation material which form the test sample 3 may be determined.

The electrode system 1 comprises a high voltage connection 5 connected to a high voltage unit. The electrode system 1 comprises a measurement connection 7 connected to measurement equipment, such as a galvanometer or an electrometer.

Additionally, the electrode system 1 comprises a high voltage electrode 9 electrically connected to the high voltage connection 5, and a measurement electrode 11 electrically connected to the measurement connection 7.

The electrode system 1 may comprise a guard electrode 13. The guard electrode 13 is connected to earth and may be arranged concentrically with the measurement electrode 11.

The test sample 3 is arranged between and in contact with the high voltage electrode 9 and the measurement electrode 11.

Leakage current through the test sample 3 can thereby be measured by the measurement equipment. The conductivity of the test sample 3 can thus be determined.

The test sample 3 is composed of an insulation plate 3a consisting of the polymeric electrical insulation material, and the foreign material 3b.

A method of determining the compatibility between the polymeric electrical insulation material and the foreign material 3b will now be described in more detail with reference to the flowchart in Fig. 2.

In a step a) the test sample is prepared. The test sample 3 is prepared from the insulating plate 3a consisting of the polymeric electrical insulation material and the foreign material 3b.

The insulating plate 3a and the foreign material 3b are preferably arranged in direct contact with each other. The foreign material 3b may be arranged in direct contact with the insulating plate 3a at one side of the insulating plate 3a only, or on both sides of the insulating plate 3b, such that the insulating plate 3a is sandwiched between the foreign material 3b, as shown in Fig. 1. Alternatively, the insulating plate 3a and the foreign material 3b may be arranged in indirect contact with each other.

The insulating plate 3a may for example be 0.5-2 mm thick.

The polymeric electrical insulation material may for example comprise polyethylene such as XLPE, polypropylene, EPDM or EPR. The polymeric electrical insulation material may be a DC insulation material, for example designed for voltages higher than 300 kV.

The foreign material 3b may for example be a polymeric material, a lubricating material such as grease, a metal such as lead, copper, a copper alloy, aluminium, an aluminium alloy, or a stainless steel type, or a semiconductive material. The foreign material 3b may according to one example be a tape such as a conductor tape or a swelling tape, i.e., a water swellable tape.

In one example, the insulating plate 3a may be prepared the same way as would the insulation layer of a high voltage power cable. The insulating plate 3b may thus for example be crosslinked and degassed following the crosslinking.

In one example, the insulating plate 3a and the foreign material 3b may be pressed together. The insulating plate 3a and the foreign material 3b may be pressed together in a press moulding machine. The thus obtained press moulded test sample 3 may comprise a single layer foreign 3a material directly contacting the insulating plate 3b, or the press moulded test plate 3 may comprise the insulating plate 3a arranged in direct contact with and between two layers of the foreign material 3b in a sandwiched configuration.

The test sample 3 may be subjected to a heat treatment. Thus, the insulating plate 3a and the foreign material 3b may be heat treated when they are in direct or indirect contact. The heat treatment may involve heating the test sample 3 at a temperature in a range of 70-110°C. In one example, the heat treatment involves subjecting the test plate 3 to temperature cycles between room temperature and the range of 70-110°C.

The prepared test sample 3 is placed in the electrode system/conductivity cell 1.

In a step b) a voltage is applied over the test sample 3. In step b) the electric field over the test sample 3 may be in a range of 15-50 kV/mm.

The voltage may as an example be applied over the test sample 3 for 24h in step b)

In one example, the thickness of the test plate 3 may be 1 mm, and the voltage applied may be 30 kV/mm.

In a step c) a conductivity of the test sample 3 is determined based on measurements taken during step b), i.e., while the voltage is applied over the test sample 3. The leakage current through the test sample 3 may thus be determined. The volume resistivity may be determined from the leakage current, and the conductivity of the test sample 3 may be determined based on the volume resistivity.

In a step d) the conductivity of the test sample 3 is compared with a reference conductivity. The reference conductivity may be the conductivity of a non-contaminated reference sample of the polymeric electrical insulation material that is being tested. Thus, the reference sample is a piece consisting of an insulation plate of the same polymeric electrical insulation material as the one that is currently being evaluated.

In a step e) it is concluded whether the foreign material 3b is compatible with the polymeric electrical insulation material based on the comparison in step d). The foreign material 3b is compatible with the polymeric electrical insulation material in case the conductivity determined in step c) deviates with at most a predetermined amount from the reference conductivity. Especially, if the conductivity determined in step c) is equal to or higher than the reference conductivity but the deviation is smaller than or equal to a predetermined threshold value, then it is concluded that the foreign material 3b is compatible with the polymeric electrical insulation material. Otherwise, it is concluded that the foreign material 3b is non-compatible with the polymeric electrical insulation material.

Steps d) and/or e) may be computer-implemented, carried out e.g., by a processing circuitry or they may be carried out manually, for example by a test operator.

Steps a)-e) can be repeated for a plurality of different test samples 3, each comprising the same polymeric electrical insulation material as the insulation plate 3a but different foreign material 3b. For example, the foreign material may be many different types of tapes, such as different types of conductor tape or different types of swelling tape, to find conductor tape or swelling tape candidates that are compatible with the polymeric electrical insulation material. Thus, many test samples 3 can be prepared, each comprising the same polymeric electrical insulation, but each having a different foreign material 3 of the same general type, e.g., conductor tape or swelling tape. Further, such tests can be carried out for a plurality of different components of a high voltage power cable, such as for lubricating material, metallic water blocking barrier, and so on. For each component to be tested for compatibility with the polymeric electrical insulation material a plurality of test samples may be prepared.

Once compatible foreign materials have been found based on the method, a high voltage power cable may be built using the polymeric electrical insulation material for the insulation layer and the compatible foreign materials in e.g., the conductor tape, the swelling tape, and so on, for the other components of the high voltage power cable.

The high voltage power cable thus built may be a submarine power cable or an underground power cable.

The inventive concept has mainly been described above with reference to a few examples. However, as is readily appreciated by a person skilled in the art, other embodiments than the ones disclosed above are equally possible within the scope of the inventive concept, as defined by the appended claims.

## Claims

1. Method of determining compatibility between a polymeric electrical insulation material for an insulation layer of a high voltage power cable and a foreign material (3b), the method comprising:
a) preparing a test sample (3) from A) an insulation plate (3a) consisting of the polymeric electrical insulation material, and B) the foreign material (3b),
b) applying a voltage over the test sample (3),
c) determining a conductivity of the test sample (3) based on measurements taken during step b),
d) comparing the conductivity of the test sample (3) with a reference conductivity, and
e) concluding whether the foreign material (3b) is compatible with the polymeric electrical insulation material based on the comparison in step d).

2. Method as claimed in claim 1, wherein step e) involves concluding that the foreign material (3b) is compatible with the polymeric electrical insulation material in case the conductivity deviates with at most a predetermined amount from the reference conductivity, and otherwise concluding that the foreign material (3b) is non-compatible with the polymeric electrical insulation material.

3. Method as claimed in claim 1 or 2, steps b) and c) are performed in a conductivity cell.

4. Method as claimed in any of the preceding claims, wherein in step a) the insulation plate (3a) is set in direct contact with the foreign material (3b).

5. Method as claimed in claim 4, wherein step a) further involves pressing together the insulation plate (3a) and the foreign material (3b) in a press moulding machine.

6. Method as claimed in claim 4, wherein step a) involves heat treating the insulation plate (3a) and the foreign material (3b) when they are in direct contact.

7. Method as claimed in claim 6, wherein the heat treatment involves heating at a temperature in the range of 70-110°C.

8. Method as claimed in claim 7, wherein the heat treatment involves subjecting the insulation plate (3a) and the foreign material (3b) to temperature cycles between room temperature and said temperature in the range of 70-110°C.

9. Method as claimed in any of the preceding claims, wherein the reference conductivity is the conductivity of a non-contaminated reference sample of the polymeric electrical insulation material.

10. Method as claimed in any of the preceding claims, wherein an electric field over the test sample (3) in step b) is in a range of 15-50 kV/mm.

11. Method as claimed in any of the preceding claims, wherein the insulation plate (3a) has a thickness in a range of 0.5-2 mm.

12. Method as claimed in any of the preceding claims, wherein the foreign material is one of a polymeric material, a lubricating material, a metal, and a semiconductive material.

13. Method as claimed in any of claims 1-11, wherein the foreign material is a conductor tape or a swelling tape.

14. Method as claimed in any of the preceding claims, wherein the polymeric electrical insulation material is a DC insulation material.

15. Method as claimed in claim 14, wherein the DC insulation material is designed for voltages greater than 300 kV.

## Patentansprüche

1. Verfahren zur Bestimmung der Kompatibilität zwischen einem elektrischen Polymerisoliermaterial für eine Isolierschicht eines Hochspannungsstromkabels und einem Fremdmaterial (3b), wobei das Verfahren Folgendes umfasst:
a) Vorbereitung einer Testprobe (3) aus A) einer Isolierplatte (3a), die aus dem elektrischen Polymerisoliermaterial besteht, und B) dem Fremdmaterial (3b),
b) Anlegung einer Spannung an die Testprobe (3),
c) Bestimmung einer Leitfähigkeit der Testprobe (3) anhand der Messungen, die in Schritt b) durchgeführt werden,
d) Vergleich der Leitfähigkeit der Testprobe (3) mit einer Referenzleitfähigkeit und
e) Bewertung, ob das Fremdmaterial (3b) mit dem elektrischen Polymerisoliermaterial anhand des Vergleichs in Schritt d) kompatibel ist.

2. Verfahren nach Anspruch 1, wobei Schritt e) die Bewertung einschließt, dass das Fremdmaterial (3b) mit dem elektrischen Polymerisoliermaterial kompatibel ist, wenn die Leitfähigkeit höchstens um einen vorbestimmten Betrag von der Referenzleitfähigkeit abweicht, und andernfalls die Bewertung, dass das Fremdmaterial (3b) nicht mit dem elektrischen Polymerisoliermaterial kompatibel ist.

3. Verfahren nach Anspruch 1 oder 2, wobei die Schritte b) und c) in einer Leitfähigkeitszelle durchgeführt werden.

4. Verfahren nach einem der vorhergehenden Ansprüche, wobei in Schritt a) die Isolierplatte (3a) in direkten Kontakt mit dem Fremdmaterial (3b) gebracht wird.

5. Verfahren nach Anspruch 4, wobei Schritt a) ferner die Zusammenpressung der Isolierplatte (3a) und des Fremdmaterials (3b) in einer Pressformmaschine einschließt.

6. Verfahren nach Anspruch 4, wobei Schritt a) die Wärmebehandlung der Isolierplatte (3a) und des Fremdmaterials (3b) einschließt, wenn diese in direktem Kontakt stehen.

7. Verfahren nach Anspruch 6, wobei die Wärmebehandlung eine Erwärmung auf eine Temperatur im Bereich von 70-110 °C einschließt.

8. Verfahren nach Anspruch 7, wobei die Wärmebehandlung einschließt, die Isolierplatte (3a) und das Fremdmaterial (3b) Temperaturzyklen zwischen der Raumtemperatur und der Temperatur im Bereich von 70-110 °C zu unterziehen.

9. Verfahren nach einem der vorhergehenden Ansprüche, wobei die Referenzleitfähigkeit die Leitfähigkeit einer nicht kontaminierten Referenzprobe des elektrischen Polymerisoliermaterials ist.

10. Verfahren nach einem der vorhergehenden Ansprüche, wobei ein elektrisches Feld über der Testprobe (3) in Schritt b) in einem Bereich von 15-50 kV/mm liegt.

11. Verfahren nach einem der vorhergehenden Ansprüche, wobei die Isolierplatte (3a) eine Dicke in einem Bereich von 0,5-2 mm aufweist.

12. Verfahren nach einem der vorhergehenden Ansprüche, wobei das Fremdmaterial eines der folgenden Materialien ist: ein Polymermaterial, ein Schmiermaterial, ein Metall und ein Halbleitermaterial.

13. Verfahren nach einem der Ansprüche 1-11, wobei das Fremdmaterial ein Leiterband oder ein Quellband ist.

14. Verfahren nach einem der vorhergehenden Ansprüche, wobei das elektrische Polymerisoliermaterial ein Gleichstromisoliermaterial ist.

15. Verfahren nach Anspruch 14, wobei das Gleichstromisoliermaterial für Spannungen von mehr als 300 kV ausgelegt ist.

## Revendications

1. Procédé de détermination de compatibilité entre un matériau d'isolation électrique polymère pour une couche d'isolation d'un câble d'alimentation haute tension et un matériau étranger (3b), le procédé comprenant :
a) la préparation d'un échantillon d'essai (3) A) d'une plaque d'isolation (3a) constituée du matériau d'isolation électrique polymère, et B) du matériau étranger (3b),
b) l'application d'une tension sur l'échantillon d'essai (3),
c) la détermination d'une conductivité de l'échantillon d'essai (3) sur la base de mesures prises au cours de l'étape b),
d) la comparaison de la conductivité de l'échantillon d'essai (3) à une conductivité de référence, et
e) la conclusion si le matériau étranger (3b) est compatible avec le matériau d'isolation électrique polymère sur la base de la comparaison de l'étape d).

2. Procédé selon la revendication 1, dans lequel l'étape e) implique la conclusion que le matériau étranger (3b) est compatible avec le matériau d'isolation électrique polymère dans le cas où la conductivité s'écarte de la conductivité de référence d'au plus une quantité prédéterminée, sinon la conclusion que le matériau étranger (3b) n'est pas compatible avec le matériau d'isolation électrique polymère.

3. Procédé selon la revendication 1 ou 2, dans lequel les étapes b) et c) sont réalisées dans une cellule de conductivité.

4. Procédé selon l'une quelconque des revendications précédentes, dans lequel, à l'étape a), la plaque d'isolation (3a) est mise en contact direct avec le matériau étranger (3b).

5. Procédé selon la revendication 4, dans lequel l'étape a) implique en outre la pression de la plaque d'isolation (3a) et du matériau étranger (3b) l'un contre l'autre dans une machine de moulage par pression.

6. Procédé selon la revendication 4, dans lequel l'étape a) implique le traitement thermique de la plaque d'isolation (3a) et du matériau étranger (3b) lorsqu'ils sont en contact direct.

7. Procédé selon la revendication 6, dans lequel le traitement thermique implique le chauffage à une température dans la plage de 70 à 110 °C.

8. Procédé selon la revendication 7, dans lequel le traitement thermique implique la soumission de la plaque d'isolation (3a) et du matériau étranger (3b) à des cycles de température entre la température ambiante et ladite température dans la plage de 70 à 110 °C.

9. Procédé selon l'une quelconque des revendications précédentes, dans lequel la conductivité de référence est la conductivité d'un échantillon de référence non contaminé du matériau d'isolation électrique polymère.

10. Procédé selon l'une quelconque des revendications précédentes, dans lequel un champ électrique sur l'échantillon d'essai (3) à l'étape b) est dans une plage de 15 à 50 kV/mm.

11. Procédé selon l'une quelconque des revendications précédentes, dans lequel la plaque d'isolation (3a) présente une épaisseur dans une plage de 0,5 à 2 mm.

12. Procédé selon l'une quelconque des revendications précédentes, dans lequel le matériau étranger est l'un parmi un matériau polymère, un matériau lubrifiant, un métal et un matériau semi-conducteur.

13. Procédé selon l'une quelconque des revendications 1 à 11, dans lequel le matériau étranger est un ruban conducteur ou une bande de gonflement.

14. Procédé selon l'une quelconque des revendications précédentes, dans lequel le matériau d'isolation électrique polymère est un matériau d'isolation CC.

15. Procédé selon la revendication 14, dans lequel le matériau d'isolation CC est conçu pour des tensions supérieures à 300 kV.
